# EUROPÄISCHE PATENTANMELDUNG

(11) **EP 4 778 479 A1**
(43) Veröffentlichungstag der Anmeldung: **22.07.2026**
(21) Anmeldenummer: 25152594.5
(22) Anmeldetag: 17.01.2025
(51) Int. Cl.: A61B 18/12

(54) **MEDIZINISCHER GENERATOR MIT NETZAUSFALLERKENNUNG**

(71) Anmelder: Erbe Elektromedizin GmbH, 72072 Tübingen (DE)
(72) Erfinder: Egler, Thomas, 72770 Reutlingen (DE); Ripplinger, Thomas, 72810 Gomaringen (DE)
(74) Vertreter: Rüger Abel Patentanwälte PartGmbB

(57) **Zusammenfassung**

Mit dem erfindungsgemäßen Konzept können beispielsweise während des Betriebs vom Netz getrennte Generatoren (11) bedenkenlos ohne gesonderte Vorsichtsmaßnahme wieder mit dem Netz verbunden werden. Beispielsweise können während des Betriebs gezogene Gerätestecker wieder in das Gerät eingesteckt oder während des Betriebs betätigte Hauptschalter wieder eingeschaltet werden. Die Steuereinrichtung (14) kann dann auf die im geordneten Abschaltbetrieb gespeicherten Daten und Messwerte und Einstellungen zurückgreifen.

## Beschreibung

Die Erfindung betrifft einen medizinischen Generator, der eine Netzüberwachungseinrichtung aufweist.

Aus der EP 2 853 217 B1 ist der Grundaufbau eines chirurgischen Generators bekannt. Dieser weist eingangsseitig einen Gleichrichter und eine Leistungsfaktor-Korrekturschaltung auf, die einen Gleichstrom-Zwischenkreis speist, an den ein Gleichspannungs-Umsetzer angeschlossen ist. Die Leistungsfaktor-Korrekturschaltung und der Gleichspannungs-Umsetzer bilden zusammen eine Stromversorgungseinheit für den Generator. Die Leistungsfaktor-Korrekturschaltung kommuniziert über eine Datenschnittstelle mit einer Systemsteuereinrichtung, um schnelle Leistungsanpassungen vornehmen zu können.

Weiterer Stand der Technik geht aus der EP 2 475 319 B1 hervor.

Neben der Stromversorgungseinheit weisen medizinische Generatoren eine Funktionsbaugruppe auf, die beispielsweise einen Hochfrequenzoszillator zur Erzeugung leistungsstarker HF-Ströme umfasst, mit denen ein oder mehrere Instrumente zur Bewirkung chirurgischer Effekte an einem Patienten gespeist werden können. Die Steuerung der Funktionsbaugruppe und gegebenenfalls auch der Stromversorgungseinheit unterliegt der Systemsteuereinrichtung, die typischerweise programmgesteuert arbeitet.

Generatoren dieser Bauart sind weit verbreitet. Bei unvorhergesehenem Stromausfall, unbeabsichtigter Netztrennung, zum Beispiel durch Ziehen des Netzsteckers, sonstige Unterbrechung der Stromzufuhr oder auch versehentliches Abschalten während des Betriebs, können in der Systemsteuerung Daten verloren gehen und unerwünschte Zustände auftreten.

Davon ausgehend ist es Aufgabe der Erfindung, einen netzausfallsicheren medizinischen Generator zu schaffen.

Diese Aufgabe wird mit dem medizinischen Generator nach Anspruch 1 gelöst.

Der erfindungsgemäße medizinische Generator weist eine Netzüberwachungseinrichtung auf, die eingangsseitig mit dem Netzspannungseingang verbunden ist und eine Auswerteschaltung aufweist, die das Vorhandensein der Netzspannung überwacht. Sie ist darauf eingerichtet, bei Wegfall der Netzspannung während des Betriebs ein Abschaltsignal zu erzeugen, das geeignet ist, die Funktionsbaugruppe abzuschalten, um diese in einen sicheren Zustand zu überführen, bevor die Stromversorgungseinheit nicht mehr in der Lage ist, die Funktionsbaugruppe aus dem Speicher und somit aus der darin gespeicherten Energie mit Strom zu versorgen. Das Abschalten der Funktionsbaugruppe und die Überführung in einen sicheren Zustand kann das Schließen von Medienventilen, gegebenenfalls in einer vorgegebenen Sequenz, das Sichern von Daten, z.B. Sensordaten, oder Daten aus dem Ablauf eines in einer Steuereinrichtung laufenden Steuerprogramms (z.B. über die bereits durchlaufene Behandlungsdauer oder dergleichen) sein. Beispielsweise kann bei einem Generator zur Argonplasmakoagulation vorgesehen sein, dass bei Empfang eines Abschaltsignals sofort der Oszillator abgeschaltet wird, der das Instrument mit Strom speist, wohingegen das Ventil zur Speisung des Instruments mit Argon erst danach geschlossen wird. Vielfältige andere Abschaltsequenzen sind möglich.

Der Speicher kann einen oder mehrere Speicherkondensatoren (z.B. Elektrolytkondensatoren) umfassen, die an einer oder an mehreren Stellen der Schaltung des Generators, insbesondere in der Stromversorgungseinheit, angeordnet sind. Die Stromversorgungseinheit kann zum Beispiel eine Leistungsfaktor-Korrekturschaltung aufweisen, die einen Gleichstrom-Zwischenkreis speist, an den ein oder mehrere Kondensatoren angeschlossen sind. An den Gleichspannungs-Zwischenkreis können ein oder mehrere Gleichspannungsumsetzer angeschlossen sein, die ausgangsseitig ebenfalls mit Kondensatoren oder Kondensatorgruppen versehen sein können. Der Begriff "Speicher" bezieht dabei diejenigen Kondensatoren oder Kondensatorgruppen ein, aus denen die bei einem Netzausfall in einen sicheren Zustand zu überführenden Baugruppe zu speisen ist. Diese Baugruppe kann die gesamte Funktionsbaugruppe oder auch lediglich ein Teil derselben, beispielsweise die Steuereinrichtung sein, die Teil der Funktionsbaugruppe sein kann.

Die Stromversorgungseinheit ist dank ihres Speichers in der Lage, die Funktionsbaugruppe bei stromlosem Netzspannungseingang über eine Versorgungszeitspanne von zum Beispiel 100 Millisekunden (ms) oder auch länger mit Betriebsstrom zu versorgen. Die Versorgungszeitspanne ist durch entsprechende Dimensionierung des Speichers mindestens so groß, dass die Funktionsbaugruppe, und insbesondere deren Steuerung, innerhalb der Versorgungszeitspanne in einen sicheren Abschaltzustand überführbar ist. Laufende Programme können geordnet beendet und Daten, zum Beispiel Sensordaten, Betriebsdaten oder dergleichen, gesichert werden.

Vorzugsweise ist die Netzüberwachungseinrichtung dazu eingerichtet, das Abschaltsignal erst nach einer festgelegten Karenzzeitspanne zu erzeugen, wobei die Karenzzeitspanne geringer ist als die Versorgungszeitspanne. Dabei ist die Differenz aus der Versorgungszeitspanne und der Karenzzeitspanne größer als die für die Überführung der Funktionsbaugruppe oder deren Steuerung erforderliche Abschaltzeitspanne.

Durch die Festlegung der Karenzzeitspanne kann verhindert werden, dass die Netzüberwachungseinrichtung aufgrund belangloser Netzstörungen ein Abschaltsignal erzeugt. Der Generator ist somit tolerant gegen kleinere, an sich unschädliche Netzstörungen. Solche belanglosen und somit tolerierbaren Netzstörungen können beispielsweise der vollständige oder teilweise Wegfall von Netzhalbwellen oder sonstige Netzstörungen sein, beispielsweise kurzzeitige Spannungsschwankungen. Beträgt die Karenzzeitspanne beispielsweise 10 oder etwas mehr als 10 Millisekunden, führt der Wegfall einer gesamten Netzhalbwelle noch nicht zur Erzeugung eines Abschaltsignals. Fällt jedoch die Netzspannung gänzlich aus, wird nach Ablauf der Karenzzeitspanne das Abschaltsignal erzeugt und die Steuerung der Funktionsbaugruppe in einen sicheren Zustand überführt. Der Netzspannungsausfall kann ein allgemeiner Stromausfall sein. Er kann auch durch unbeabsichtigtes Ziehen des Netzsteckers des Generators (zum Beispiel durch Verschieben des Generators im OP-Saal) oder durch unbeabsichtigte Betätigung des Netzhauptschalters des Generators verursacht sein.

Die zur Steuerung der Funktionsbaugruppe und des gesamten Generators vorgesehene Steuereinrichtung kann einen (oder mehrere) Controller (Rechnerbausteine) umfassen, der bzw. die zur Abarbeitung eines Steuerprogramms eingerichtet sind und von dem Steuerprogramm gesteuert arbeiten. Dieses Steuerprogramm ist dazu eingerichtet die Lieferung von Spannung, Strom oder auch Medien, wie zum Beispiel Gas oder Flüssigkeiten, an angeschlossene Instrumente zu steuern und dabei die gewünschten Operationsmodes zu realisieren. Operationsmodes bestimmen die chirurgische, am Patienten zu erzielende Wirkung. Für jeden Mode sind bestimmte elektrische Eigenschaften des zu dem Instrument geleiteten Stroms vordefiniert und nur in vorgegebenen Bereichen variierbar. Solche elektrischen Eigenschaften sind z.B. die Spannung, die Frequenz, die Modulationsart, das Puls-Pause-Verhältnis bei Ein/Aus-Modulation, Grenzen für die Stromstärke, Leistungslimitierungen, zeitliche Verläufe der genannten Größen und dergleichen mehr.

Das Steuerprogramm ist dazu eingerichtet, bei Empfang eines Abschaltsignals eine Abschaltsequenz einzuleiten und abzuarbeiten. Im Rahmen der Abschaltsequenz kann der Betrieb der Funktionsbaugruppe beendet werden, gewonnene Sensordaten und gegebenenfalls sonstige Daten gesichert werden und laufende Programme, falls nötig, beendet werden. Durch das Abspeichern der Daten kann das Steuerprogramm bei Wiederkehr der Netzspannung seinen Betrieb geordnet fortsetzen. Zum Beispiel kann das Steuerprogramm dazu eingerichtet sein, bei Empfang des Abschaltsignals im Rahmen der Abschaltsequenz Nutzereingaben und/oder Programmdaten, z.B. über den eingestellten Mode, abzuspeichern. So bleibt der eingestellte Mode über den Netzspannungsausfall hinweg erhalten. Innerhalb des Modes aufgezeichnete Behandlungsdaten, zum Beispiel über Anwendungsdauer oder Anwendungsintensität, bleiben ebenfalls erhalten, sodass der Betrieb der Funktionsbaugruppe nach Wiederkehr der Netzspannung in für den Patienten ungefährliche Weise fortsetzbar ist.

Die Abschaltsequenz kann auch vorsehen, dass sofort nach Empfang des Abschaltsignals leistungsstarke Verbraucher, wie z.B. der Oszillator zur Speisung chirurgischer Instrumente, abgeschaltet werden, so dass die im Speicher vorhandene Energie vorwiegend zur weiteren Speisung der Systemsteuerung oder einer sonstigen Steuereinrichtung genutzt werden kann. Dadurch wird es erleichtert, die Steuereinrichtung (insbesondere die gesamte Systemsteuerung) geordnet abzuschalten und alle nötigen Daten zu sichern, ohne dafür gesonderte Energiespeicher vorsehen zu müssen.

Die Netzüberwachungseinrichtung ist vorzugsweise über einen Vollweggleichrichter mit dem Netzspannungseingang verbunden, sodass das Vorhandensein beider Halbwellen der Netzspannung überwacht wird. Zwischen dem Netzanschluss und dem Vollweggleichrichter kann ein Netzfilter mit einem oder mehreren reaktiven Bauelementen, z.B. miteinander koppelnden oder nichtkoppelnden Induktoren und insbesondere Kondensatoren angeordnet sein, um die Fortpflanzung von elektrischen Störungen aus dem Generator in das Netz und aus dem Netz in den Generator zu verhindern.

Die Netzüberwachungseinrichtung ist darauf eingerichtet, sowohl einen niederohmigen als auch einen hochohmigen Netzspannungsausfall zu erfassen. Sie ist insbesondere darauf eingerichtet, diese verschiedenen Ausfallszenarien auch dann sicher zu erfassen, wenn in dem Netzfilter aufgrund der dort vorhandenen energiespeichernden Bauelemente bei einem hochohmigen Spannungsausfall Restspannungen vorhanden sind.

Ein niederohmiger Netzspannungsausfall liegt vor, wenn die Netzspannung auf Null zusammenbricht, sodass die beiden Netzleitungen praktisch auf gleichem Potenzial liegen ("black out"), oder wenigstens soweit vermindert ist, dass ein ordnungsgemäßer Betrieb des Generators nicht mehr möglich ist ("brown out"). Ein hochohmiger Netzspannungsausfall liegt vor, wenn beispielsweise der Netzstecker unbeabsichtigt aus der Steckdose oder der Gerätestecker aus dem Generator gezogen wird. Dabei können, zumindest kurzzeitig, am Netzeingang der Stromversorgungseinheit und somit am Eingang der Netzüberwachungseinrichtung Gleichspannungsanteile anliegen, die beispielsweise von geladenen Kondensatoren des eingangsseitigen Netzfilters herrühren.

Beide Fehlerszenarien werden von der Netzüberwachungseinrichtung erfasst. Diese weist dazu einen an den eingangsseitigen Gleichrichter angeschlossenen Hochpass auf, der keine Gleichspannung passieren lässt. Im Weiteren kann die Netzüberwachungseinrichtung Impulsformer sowie eine Zeitgeberschaltung aufweisen, die die Karenzzeitspanne bestimmt. Die Zeitgeberschaltung kann als retriggerbares Monoflop ausgebildet sein, welches das Abschaltsignal erzeugt, falls es nicht innerhalb der Karenzzeitspanne von der Netzseite her durch einen neuen Impuls getriggert wird. Diese Impulse werden von jeder Netzhalbwelle neu erzeugt.

Weitere Einzelheiten vorteilhafter Ausführungsformen der Erfindung sind Gegenstand von Unteransprüchen sowie der Beschreibung und der zugehörigen Zeichnung. In der Zeichnung zeigen:
Figur 1 einen erfindungsgemäßen medizinischen Generator mit Netzausfallerkennung, in schematisierter Darstellung,
Figur 2 die Stromversorgungseinheit des Generators nach Figur 1, als Prinzipschaltbild,
Figur 3 die Stromversorgungseinheit, in schematisierter und vereinfachter Darstellung sowie die zugehörige Netzüberwachungseinrichtung,
Figur 4 den zeitlichen Verlauf der gleichgerichteten Netzwechselspannung in einem beispielhaften Diagramm und
Figur 5 den Verlauf der Versorgungsspannung und des Abschaltsignals im zeitlichen Verlauf entsprechend der Netzspannung nach Figur 4 als Diagramm.

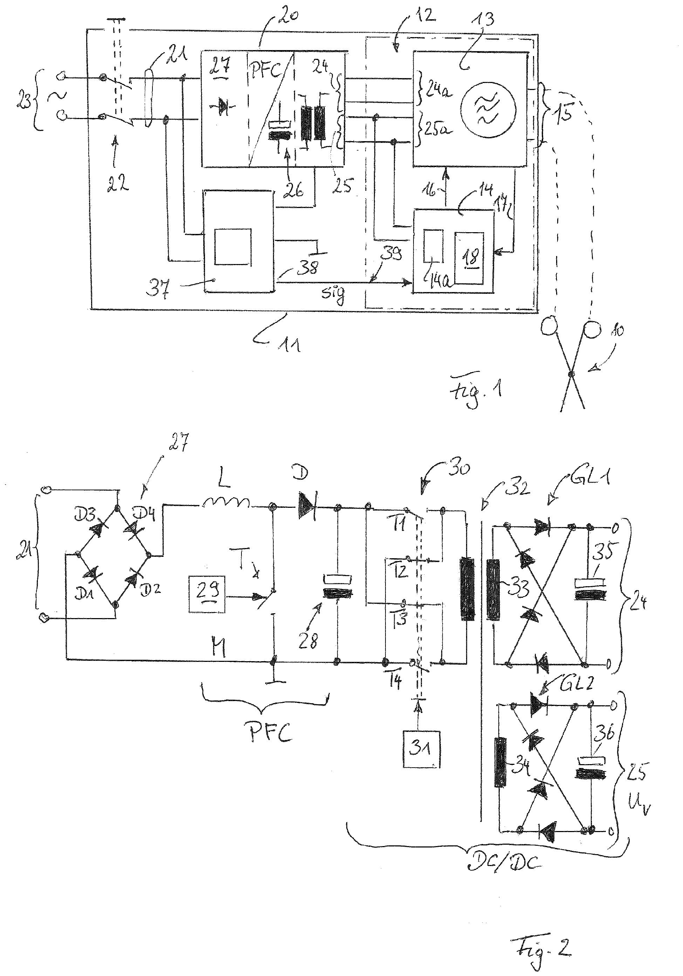

In Figur 1 ist ein Instrument 10 zur Behandlung eines menschlichen oder tierischen Patienten sowie ein Generator 11 schematisch veranschaulicht, der zur Speisung des Instruments 10 mit Strom dient. Das Instrument 10 kann ein monopolar oder bipolar oder multipolar ausgebildetes elektrochirurgisches Instrument sein. Es kann beispielsweise zum Koagulieren, Schneiden, Fusionieren von Gewebe oder zur Durchführung anderer elektrochirurgischer Eingriffe dienen. Der Generator 11 kann außer der Versorgung mit Strom für elektrochirurgische Eingriffe auch zur Versorgung des Instruments 10 mit anderen Medien, beispielsweise Flüssigkeiten, Dämpfen, Aerosolen oder Gasen, beispielsweise Argon, eingerichtet sein. Der Generator kann auch eine Absaugeeinrichtung umfassen, z.B. um Gase, Rauch oder Flüssigkeiten aus einem Operationsgebiet abzusaugen. Das Instrument 10 kann dann ein Instrument zur Argon-Plasma-Koagulation oder auch ein kryochirurgisches Instrument oder dergleichen sein.

Zur Speisung des Instruments 10 umfasst der Generator 11 eine Funktionsbaugruppe 12, die eines oder mehrere der genannten Medien (Strom, Gas, Flüssigkeit) für das Instrument 10 bereitstellt. In Figur 1 ist die Funktionsbaugruppe 12 beispielshaft lediglich als rein elektrische Versorgungsbaugruppe mit einem Hochfrequenzoszillator 13 und einer Steuereinrichtung 14 veranschaulicht. Die Steuereinrichtung kann auch dazu eingerichtet sein, den gesamten Generator zu steuern und somit eine Systemsteuerung bilden. Die Funktionsbaugruppe 12 kann aber zusätzlich oder alternativ auch weitere oder andere Versorgungsbaugruppen, z.B. eine Quelle für Kryofluide, Spülfluide oder sonstiges aufweisen.

Der Oszillator 13 weist mindestens einen Ausgang 15 auf, an den das Instrument 10 angeschlossen ist, um mit einer für die Durchführung des gewünschten chirurgischen Zwecks geeigneten Hochspannung und entsprechendem Strom versorgt zu werden. Es handelt sich dabei vorzugsweise um hochfrequenten Wechselstrom mit einer Frequenz oberhalb 200 kHz, jedoch, zumindest vorzugsweise, unterhalb 5 MHz. Zur Erzeugung der HF-Spannung kann der HF-Oszillator 13 jede bekannte Schaltungskonfiguration aufweisen.

Der HF-Oszillator 13 (sowie auch jede sonstige Versorgungsbaugruppe) arbeitet unter der Kontrolle der Steuereinrichtung 14, die dazu ausgebildet ist, Eigenschaften des von dem HF-Oszillator 13 erzeugten Stroms bzw. der Spannung vorzugeben. Solche Eigenschaften können beispielsweise die Höhe der Spannung und/oder des Stroms, die abgegebene Leistung, die Art der dem Strom aufgeprägten Modulation und die Modulationstiefe, der Crestfaktor und anderes sein. Damit kann die Steuereinrichtung 14 den Operationsmodus z.B. Koagulation, Schnitt, Gewebefusion oder dergleichen vorgeben. Außerdem können in oder an dem HF-Oszillator 13 Sensorbaugruppen zur Gewinnung von Daten vorgesehen sein, z.B. über die Größe der an das Instrument abgegebenen Spannung, des Stroms, der Behandlungsdauer, der Leistung, des Gewebewiderstands und dergleichen mehr. Diese Sensorbaugruppen können entsprechende Daten an die Steuereinrichtung 14 weitergeben. Die Steuerung des Oszillators 13 durch die Steuereinrichtung 14 ist durch einen Pfeil 16 symbolisiert. Die Übermittlung von Daten von dem Oszillator 13 an die Steuereinrichtung 14 ist durch einen Pfeil 17 symbolisiert.

Zur Steuerung des HF-Oszillators 13 enthält die Steuereinrichtung 14 eine programmierbare Einheit, wie z.B. einen Controller 14a, eine Rechnerbaugruppe oder ein Netzwerk von Controllern oder Rechnern. Der oder die Controller 14a oder Rechner sind dazu eingerichtet, programmgesteuert zu arbeiten. Außerdem enthält die Steuereinrichtung 14 einen oder mehrere nicht weiter veranschaulichte Datenspeicher zur Aufnahme und Speicherung von einem oder mehreren Programmen, von Daten die aus der Abarbeitung der Programme herrühren, sowie zur Speicherung der von den Sensorbaugruppen gelieferten Daten (Pfeil 17).

Die Steuereinrichtung 14 weist außerdem eine Ein- und Ausgabeeinrichtung 18 auf, die Bedienelemente für Nutzereingaben und Ausgabeelemente enthält. Bei den Eingabeelementen kann es sich um Tasten, Knöpfe, Touchscreens oder dergleichen handeln. Bei den Ausgabemitteln kann es sich um Bildschirme zur Wiedergabe optischer Informationen, um Kontrolllampen, Messinstrumente und dergleichen handeln. Die Ein- und Ausgabemittel können jedoch auch außerhalb des Generators 10 angeordnet und z.B. durch ein tragbares Gerät gebildet sein, dass über eine drahtgebundene oder drahtlose Datenverbindung mit dem Generator 10 verbunden ist.

Die Funktionsbaugruppe 12 ist an eine Stromversorgungseinheit 20 angeschlossen, deren Netzspannungseingang 21 über einen zumindest optional vorgesehenen Netzschalter 22 mit einem allgemeinen elektrischen Versorgungsnetz 23 angeschlossen ist. Der Netzschalter 22 ist dazu eingerichtet, zumindest eine, vorzugsweise aber beide zu dem Netzspannungseingang führenden Leitungen wahlweise zu schließen oder zu unterbrechen. Zwischen dem Netzschalter 22 und dem Netzspannungseingang 21 ist typischerweise ein Netzfilter vorgesehen. Zur Verbindung zwischen dem Generator 10 und dem Versorgungsnetz 23 dient ein Anschlusskabel, z.B. ein handelsübliches Kaltgerätekabel mit einem netzseitigen Stecker und einem geräteseitigen Stecker.

Bei dem Wechselspannungsnetz handelt es sich typischerweise um ein Netz mit einer Netzfrequenz von 50 Hz oder 60 Hz und einer Effektivspannung zwischen 110 Volt und 240 Volt. Prinzipiell eignet sich die Erfindung jedoch auch zur Anwendung bei Generatoren für andere Netze.

Die Stromversorgungseinheit 20 weist mindestens einen, typischerweise aber mehrere Betriebsspannungsausgänge 24, 25 auf, um die Funktionsbaugruppe 12 an entsprechenden Betriebsspannungseingängen 24a, 25a mit den nötigen Betriebsspannungen zu versorgen. Die Betriebsspannungen sind Gleichspannungen. Beispielsweise kann an dem Betriebsspannungsausgang 24 eine Gleichspannung von mehreren 100 Volt anstehen, während an dem Betriebsspannungsausgang 25 lediglich eine für den Betrieb von Steuerungen einschließlich der Steuereinrichtung 14 geeignete Gleichspannung von zum Beispiel 3, 5, 12 oder 24 Volt anliegt. Andere Spannungen sind je nach Bauform der entsprechenden Steuerungen möglich.

Die Stromversorgungseinheit 20 ist mit ihrem Netzspannungseingang 21 über den Netzschalter 22 mit dem Versorgungsnetz 23 verbunden, um von dort her die nötige Betriebsleistung zu beziehen. Die an den Betriebsspannungsausgängen 24 und 25 anstehende Spannung ist eine vorzugsweise stabilisierte Gleichspannung - sowohl der HF-Oszillator 13 als auch die Steuereinrichtung 14 sind gleichstromgespeist.

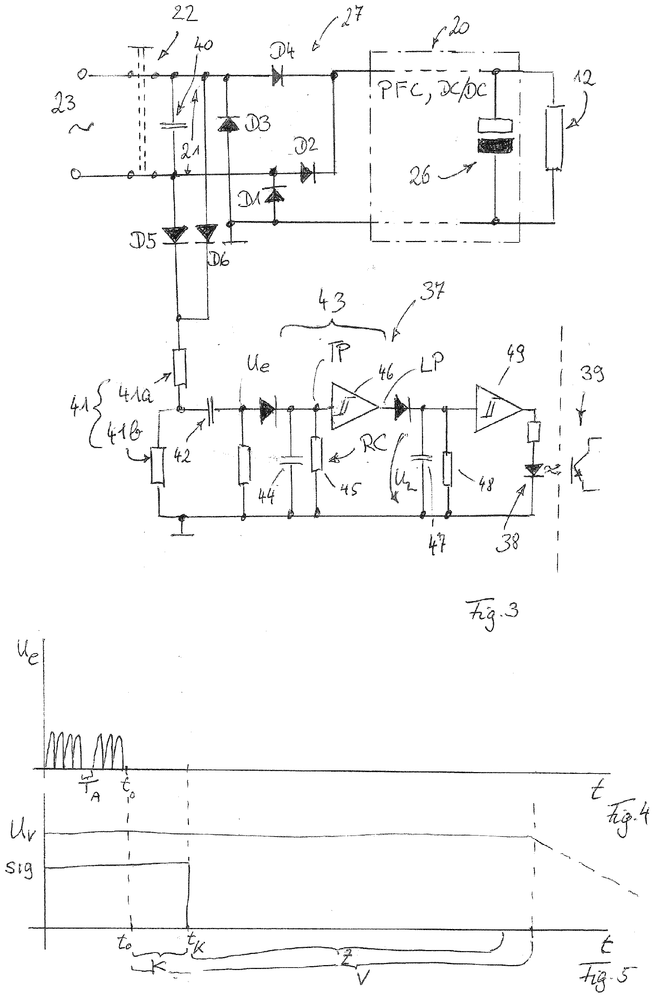

Die Stromversorgungseinheit 20 umfasst einen Speicher 26, aus dem heraus die in Figur 3 als ohmsche Last symbolisierte Funktionsbaugruppe 12, auch dann über eine Versorgungszeitspanne V (Figur 5) mit Betriebsleistung versorgbar ist, wenn die Stromversorgungseinheit 20 an ihrem Netzspannungseingang 21 keinen Strom aus dem Versorgungsnetz 23 empfängt. Eine solche Situation kann z.B. ein unerwarteter Ausfall der Netzspannung, unbeabsichtigtes oder auch bewusstes Ziehen des Netzsteckers des Generators 11 oder Betätigung des Netzschalters 22 sein. Die Versorgungszeitspanne V ergibt sich dabei aus der Kapazität des Speichers 26, der in ihm gespeicherten Ladung sowie des maximalen Leistungsbedarfs der Funktionsbaugruppe 12 während der Abarbeitung einer Abschaltsequenz.

Es kommt bei der Erfindung wesentlich darauf an, dass wenigstens die ganz oder teilweise programmgesteuerte Steuereinrichtung 14 bei Wegfall der Netzspannung über die Versorgungszeitspanne V hinweg (während der Abschaltsequenz) mit Betriebsspannung (und Strom) versorgt wird. Für den HF-Oszillator 13 ist es hingegen hinnehmbar, wenn die für ihn bereitgestellte Betriebsspannung innerhalb der Versorgungszeitspanne Z abfällt. Um jedoch keine unerwünschten chirurgischen Effekte und somit Schäden am Patienten zu verursachen, kann im Rahmen der Abschaltsequenz vorgesehen sein, dass auch die an dem Betriebsspannungsausgang 24 anstehende Spannung zum Betrieb des HF-Oszillators 13 über die Versorgungszeitspanne Z erhalten bleibt. Alternativ kann vorgesehen sein, dass der HF-Oszillator im Rahmen der Abschaltsequenz unmittelbar nach Empfang des Abschaltsignals abgeschaltet wird, um während der Abschaltsequenz möglichst wenig Energie zu verbrauchen.

Der Speicher 26 kann aus einem oder mehreren Kondensatoren, vorzugsweise Elektrolytkondensatoren bestehen, die an verschiedenen Stellen der Stromversorgungseinheit platziert sein können. Es wird dazu auf Figur 2 verwiesen, die den Grundaufbau der Stromversorgungseinheit 20 schematisch zeigt.

Die Stromversorgungseinheit 20 weist eingangsseitig einen Zweiweg-Gleichrichter 27 beispielsweise in Gestalt einer Graetzbrücke auf, an den sich eine Leistungsfaktor-Korrekturschaltung PFC anschließt. Zu dieser gehören ein Induktor L und eine Diode D, die miteinander in Reihe geschaltet sind und den vorzugsweise positiven Ausgang des Zweiweggleichrichters 27 mit dem positiven Anschluss eines Speicherkondensators 28 verbinden. Von dem Verbindungspunkt zwischen dem Induktor L und der Diode D gehen ein elektronischer Schalter T, beispielsweise in Gestalt eines Bipolartransistors, eines Feldeffekttransistors oder eines IG-BTs ab, mit seinem Emitter oder Source ebenso wie der negative Anschluss des Speicherkondensators 28 nach Masse M. Der elektronische Schalter T weist eine Steuerelektrode auf, die mit einem PFC-Taktgeber 29 verbunden ist. Durch periodisches Öffnen und Schließen des elektronischen Schalters T wird der Speicherkondensator 28 auf eine über der Spitzenspannung der Netzwechselspannung liegende Gleichspannung aufgeladen.

An die Leistungsfaktor-Korrekturschaltung PFC schließt sich ein eingangsseitig an den Speicherkondensator 28 angeschlossener Gleichspannungsumsetzer DC/DC an, der auch zur galvanischen Trennung der Betriebsspannungsausgänge 24, 25 von dem Netzspannungseingang 21 dient. Prinzipiell kann dafür jeder Gleichspannungsumsetzer bekannter Bauart eingesetzt werden. Im vorliegenden Ausführungsbeispiel umfasst der Gleichspannungsumsetzer DC/DC einen Vollbrückenwechselrichter 30 mit insgesamt vier gesteuerten elektronischen Schaltern T1, T2, T3, T4, die von einem Wechselrichter-Taktgeber 31 gesteuert sind. An den Vollbrückenwechselrichter 30 ist die Primärwicklung eines Transformators 32 angeschlossen, der eine oder mehrere Sekundärwicklungen 33, 34 aufweisen kann. Diese speisen über Zweiwege-Gleichrichter GL1, GL2 an den jeweiligen Betriebsspannungsausgängen 24, 25 angeordnete Speicherkondensatoren 35, 36.

Zur weiteren Beschreibung des dargestellten Ausführungsbeispiels wird zunächst davon ausgegangen, dass es für die geordnete Außerbetriebssetzung des Generators 11 und somit auch für das ordnungsgemäße Wiedereinschalten des Generators darauf ankommt, dass wenigstens die Steuereinrichtung 14 bei plötzlichem Netzausfall solange mit Betriebsstrom versorgt wird, bis das auf der Steuereinrichtung 14 laufende Programm geordnet beendet und die z.B. von dem HF-Oszillator 13 erhaltenen Daten sowie sonstige Daten gesichert sind. In diesem Fall wird der relevante Speicher 26 durch den Speicherkondensator 36 und den Speicherkondensator 28 gebildet. Letzterer hinsichtlich seiner Kapazität anhand des Übertragungsfaktors zwischen der Primärwicklung des Transformators 32 und der Sekundärwicklung 34 abzüglich etwaiger Wirkungsgradverluste umgerechnet. Bei Netzausfall ist der Speicher 26 geeignet, die Steuereinrichtung 14 über die Versorgungszeitspanne V hinweg, und somit solange, ggfs. länger mit Strom zu versorgen, als für die geordnete Beendigung des Betriebs der Steuereinrichtung 14 und Überführung in einen definierten Abschaltzustand nötig.

Erfindungsgemäß weist der Generator 11 eine Netzüberwachungseinrichtung 37 auf, die dazu eingerichtet ist, bei Erkennung des Wegfalls der Netzspannung an dem Netzspannungseingang 21 an einem Ausgang 38 ein Abschaltsignal sig an einen Eingang 39 der Funktionsbaugruppe 12 zu leiten. Die Funktionsbaugruppe 12 ist dabei darauf eingerichtet, bei Empfang des Abschaltsignals sig die Abschaltsequenz insbesondere der Steuereinrichtung 14 einzuleiten, die dann vorhandene Daten sichert und laufende Betriebsprogramme beendet. Dazu kann auch das Abschalten des HF-Oszillators 13 und gegebenenfalls die Abgabe optischer und/oder akustischer oder sonstiger Signale gehören, die einem Behandler signalisieren, dass der Generator 10 nicht mehr betriebsbereit ist. Auch kann signalisiert werden, dass eine eventuell begonnene chirurgische Maßnahme, beispielsweise ein Koagulationsvorgang oder ein Fusionsvorgang, zum Beispiel beim Verschließen von Gefäßen, noch nicht ordnungsgemäß abgeschlossen ist.

Die Netzüberwachungseinrichtung 37 ist aus Figur 3 ersichtlich. Die aus der Leistungsfaktor-Korrekturschaltung PFC und dem Gleichspannungsumsetzer DC/DC gebildete Stromversorgungseinheit 29 ist zusammen mit ihrem Speicher 26 lediglich schematisch veranschaulicht. Eingangsseitig ist wiederum die aus den Dioden D1 bis D4 bestehende Graetzbrücke veranschaulicht, der ein in Figur 1 lediglich durch einen Kondensator 40 veranschaulichtes Netzfilter vorgeschaltet ist.

An dem Wechselspannungseingang 21 sind außerdem Dioden D5, D6 angeschlossen, die zusammen mit den Dioden D1 und D3 der ersten Graetzbrücke (Zweiweggleichrichter 27) eine zweite Graetzbrücke, das heißt einen Zweiweggleichrichter bilden. Die so von dem Netzspannungseingang 21 abgegriffene und gleichgerichtete Spannung wird über einen aus zwei oder mehrere Widerständen 41a, 41b gebildeten Spannungsteiler 41, der der Pegelanpassung dient, und einen Kondensator 42, der der Ausfilterung von Gleichspannungsanteilen dient, einer Impulsformerschaltung 43 zugeführt. Zu der Impulsformerschaltung gehören ein Kondensator 44 und ein zu diesem parallel geschalteten Entladewiderstand 45, die ein RC-Glied bilden. An dieses RC-Glied ist eine Triggerschaltung 46 angeschlossen, die zusammen mit dem RC-Glied den Impulsformer 43 bilden.

Der Impulsformer 43 ist ausgangsseitig an einen weiteren Impulsformer mit einem weiteren RC-Glied, bestehend aus einem Kondensator 47 und einem Entladewiderstand 48, verbunden, an den wiederum eine Triggerschaltung 49 angeschlossen ist. Diese ist ausgangsseitig mit der Leuchtdiode eines Optokopplers verbunden, die den Ausgang 38 der Netzüberwachungseinrichtung 37 bildet.

Der insoweit beschriebene Generator 11 und insbesondere die Netzüberwachungseinrichtung 37 arbeiten wie folgt:

Fall 1: Die Netzspannung des Versorgungsnetzes 23 bricht während des Betriebs des Generators 11 vollständig zusammen. Damit fällt die Spannung an dem Wechselspannungseingang 21 praktisch sofort auf Null. Die Leistungsfaktor-Korrekturschaltung PFC stoppt ihren Betrieb und der Speicherkondensator 28 erhält infolgedessen keine weitere Ladung mehr. Der Gleichspannungsumsetzer DC/DC kann jedoch zumindest so lange weiterlaufen, wie die auf dem Speicherkondensator 28 vorhandene Spannung für seinen Betrieb ausreicht. So kann der Gleichspannungsumsetzer DC/DC zumindest einen Teil der Ladung des Speicherkondensators 28 auf den Kondensator 36 übertragen.

Es wird angenommen, dass der Zusammenbruch der Netzspannung zu dem in Figur 5 markierten Zeitpunkt t₀ geschieht. Zuvor, d.h. vor dem Wegfall der Netzspannung, hatte jede Netzhalbwelle über eine der Dioden D5 und D6 Ladung auf den Kondensator 44 gegeben, der von dem Entladewiderstand 45 praktisch sofort wieder entladen worden ist. Entsprechend standen an dem Ausgang der Triggerschaltung 46 netzhalbwellensynchron Ladeimpulse zur Verfügung, um das aus dem Kondensator 47 und dem Widerstand 48 gebildete RC-Glied zu laden. Solange dieses geladen blieb, hat die Triggerschaltung 49 an dem Ausgang 38 das Vorhandensein der Netzspannung signalisiert, beispielsweise indem die dort angeordnete Leuchtdiode des Optokopplers leuchtet. Die Netzüberwachungseinrichtung kann insoweit als retriggerbares Monoflop verstanden werden, das von den aus den Netzhalbwellen abgeleiteten an dem Ausgang der Triggerschaltung 46 anstehenden Impulsen stets neu getriggert worden ist. Fallen diese Impulse ab dem Zeitpunkt t₀ jedoch weg, vergeht eine gewisse Entladezeit für den Kondensator 47, nach deren Ablauf die Triggerschaltung 49 umschaltet (d.h. die Diode 38 verlischt). Das Verlöschen der Diode 38 ist das Signal sig für das Abschalten der Funktionsbaugruppe 12.

Die Zeitspanne zwischen Wegfall der Triggerimpulse zum Zeitpunkt t₀ und dem Umschalten der Triggerschaltung 49 und somit dem Verlöschen der Leuchtdiode an dem Ausgang 38 ist eine Karenzzeitspanne, die in Figur 5 mit t_{K} bezeichnet ist. Mit anderen Worten, nach einem Netzspannungsausfall wird die Karenzzeitspanne K abgewartet und das Abschaltsignal sig zum Zeitpunkt t_{K} erzeugt.

Die Festlegung der Karenzzeitspanne hat den Vorzug, dass sehr kurzzeitige Netzstörungen, wie sie in Figur 4 in der Zeitspanne T_{A} vorhanden sind, nicht zur Auslösung eines Abschaltvorgangs führen. Ein Störimpuls oder auch der Wegfall einer ganzen Netzhalbwelle führt nicht zur Erzeugung des Abschaltsignals sig. Fallen jedoch nacheinander mehrere Netzhalbwellen in einer Zeitspanne aus, deren Länge insgesamt größer als die Karenzzeitspanne K ist, wird das Signal sig erzeugt.

Die Funktionsbaugruppe 12 empfängt das Abschaltsignal sig und leitet seine Abschaltsequenz ein. Insbesondere sichert die Steuereinrichtung 14 im Rahmen der Abschaltsequenz vorhandene Daten und Programmzustände, um nach dem Wiedereinschalten einen geordneten Weiterbetrieb zu ermöglichen.

Aus der Größe des Speichers 26 und dem Strombedarf der Funktionsbaugruppe 12, oder zumindest der Steuereinrichtung 14, ergibt sich eine Versorgungszeitspanne V, über die hinweg der Betrieb der Steuereinrichtung 14 mittels der in dem Speicher 26 gespeicherten Energie sichergestellt werden kann. Die für das geordnete Abschalten insbesondere der Steuereinrichtung 14 erforderte Abschaltzeitspanne Z ist vorzugsweise geringer als (oder höchstens so groß wie) die Differenz aus der Versorgungszeitspanne V und der Karenzzeitspanne K. Dies wird durch entsprechende Bemessung der Speicherkondensatoren 28 und 36 (Figur 2) sichergestellt. In Figur 5 ist dazu veranschaulicht, dass die Versorgungsspannung U_{V} erst nach Ablauf der Versorgungszeitspanne V beginnt abzufallen.

Fall 2: Beabsichtigtes oder versehentliches Ziehen des Netzsteckers und somit Trennung des Generators 11 von dem Versorgungsnetz 23 oder auch Öffnen des Netzschalters 22 führt ebenfalls zum Wegfall der sonst von dem Netz herkommenden Versorgungsleistung. Damit werden auch in diesem Fall über die Dioden D5, D6 keine Spannungshalbwellen mehr an die Netzüberwachungseinrichtung 37 übermittelt. Der Kondensator 42 verhindert dabei auch, dass eine etwaige auf dem Kondensator 40 (d.h. in dem Netzfilter) vorhandene Gleichspannung das Vorhandensein von Netzspannung vortäuscht. Deswegen entsprechen die Vorgänge in diesem Szenario dem vorbeschriebenen Szenario insoweit vollständig.

Der erfindungsgemäße medizinische Generator weist eine Stromversorgungseinheit 20 mit Speichervermögen auf, wobei das Speichervermögen ausreicht, um den Weiterbetrieb zumindest der Steuereinrichtung 14 für eine Zeitspanne V zu ermöglichen, innerhalb derer in der Steuereinrichtung 14 vorhandene Daten, Messwerte, Programmzustände und Einstellungen und dergleichen gesichert und für einen geordneten Weiterbetrieb abgespeichert werden können. Zur Erkennung eines Netzspannungszusammenbruchs oder auch einer Trennung des Generators 11 von dem Versorgungsnetz 23 ist eine Netzüberwachungseinrichtung 37 vorgesehen, die die Netzspannung fortwährend überwacht und ein Abschaltsignal sig abgibt, sobald die Netzspannung über eine Zeitspanne nicht mehr vorhanden ist, die größer als eine Karenzzeitspanne ist. Die Karenzzeitspanne ist vorzugsweise größer als die Länge einer Netzhalbwelle, vorzugsweise größer als die Länge einer vollständigen Netzwelle.

Mit dem erfindungsgemäßen Konzept können beispielsweise während des Betriebs vom Netz getrennte Generatoren 11 bedenkenlos ohne gesonderte Vorsichtsmaßnahme wieder mit dem Netz verbunden werden. Beispielsweise können während des Betriebs gezogene Gerätestecker wieder in das Gerät eingesteckt oder während des Betriebs betätigte Hauptschalter wieder eingeschaltet werden. Die Steuereinrichtung 14 kann dann auf die im geordneten Abschaltbetrieb gespeicherten Daten und Messwerte und Einstellungen zurückgreifen.

### Bezugszeichen:

- 10: Instrument
- 11: Generator
- 12: Funktionsbaugruppe
- 13: HF-Oszillator
- 14: Steuereinrichtung
- 15: Instrumentenausgang
- 16: Pfeil
- 17: Pfeil
- 18: Ein- und Ausgabeeinrichtung
- 20: Stromversorgungseinheit
- 21: Netzspannungseingang
- 22: Netzschalter
- 23: Versorgungsnetz
- 24: Betriebsspannungsausgang
- 24a: Betriebsspannungseingang
- 25: Betriebsspannungsausgang
- 25a: Betriebsspannungseingang
- 26: Speicher
- Z: Versorgungszeitspanne
- 27: Zweiwegegleichrichter
- PFC: Leistungsfaktor-Korrekturschaltung
- L: Induktor
- D: Diode
- T: elektronischer Schalter
- 28: Speicherkondensator
- M: Masse
- 29: PFC-Steuerbaugruppe
- DC/DC: Gleichspannungsumsetzer
- 30: Vollbrückenwechselrichter
- 31: Wechselrichtertaktgeber
- 32: Transformator
- 33: erste Sekundärwicklung
- 34: zweite Sekundärwicklung
- 35: Speicherkondensator
- 36: Speicherkondensator
- 37: Netzüberwachungseinrichtung
- 38: Ausgang
- sig: Signal
- 39: Eingang
- 40: Kondensator des Netzfilters
- 41: Spannungsteiler
- 41a, 41b: Widerstände
- 42: Kondensator
- 43: Impulsformerschaltung
- 44: Kondensator
- 45: Entladewiderstand
- 46: Triggerschaltung
- 47: Kondensator
- 48: Entladewiderstand
- K: Karenzzeitspanne
- V: Versorgungszeitspanne
- Z: Abschaltzeitspanne

## Patentansprüche

1. Medizinischer Generator (11)
mit einer Stromversorgungseinheit (20), die einen an ein Wechselspannungs-Versorgungsnetz (23) anschließbaren Netzspannungseingang (21) und mindestens einen Betriebsspannungsausgang (24, 25) aufweist,
mit mindestens einer Funktionsbaugruppe (12), die mindestens einen an den Betriebsspannungsausgang (24, 25) angeschlossenen Betriebsspannungseingang (24a, 25a) und mindestens einen Instrumentenausgang (15) aufweist, an den ein chirurgisches Instrument (10) anschließbar ist,
wobei die Stromversorgungseinheit (20) einen Speicher (26) umfasst, der dazu eingerichtet ist, die Funktionsbaugruppe (12) bei stromlosem Netzspannungseingang (21) über eine festgelegte Versorgungszeitspanne (V) mit Betriebsstrom zu versorgen,
mit einer Netzüberwachungseinrichtung (37), die einen mit dem Netzspannungseingang (21) verbundenen Eingang (D5, D6) aufweist und die darauf eingerichtet ist, bei Wegfall der Netzspannung ein Abschaltsignal (sig) zu erzeugen und an die Funktionsbaugruppe (12) zu übermitteln, um diese innerhalb einer Abschaltzeitspanne (Z) in einen sicheren Zustand zu überführen.

2. Generator nach Anspruch 1, **dadurch gekennzeichnet, dass** Netzüberwachungseinrichtung (37) dazu eingerichtet ist, das Abschaltsignal (sig) erst nach einer festgelegten Karenzzeitspanne (K) zu erzeugen, wobei die Karenzzeitspanne (K) geringer ist als die Versorgungszeitspanne (V).

3. Generator nach Anspruch 2, **dadurch gekennzeichnet, dass** die Differenz aus der Versorgungszeitspanne (V) und der Karenzzeitspanne (K) größer ist, als die von der Funktionsbaugruppe (12, 14) für die Überführung in einen sicheren Zustand erforderliche Abschaltzeitspanne (Z).

4. Generator nach einem der vorstehenden Ansprüche, **dadurch gekennzeichnet, dass** die Funktionsbaugruppe (37) wenigstens einen Controller (14a) aufweist, der von einem Steuerprogramm gesteuert arbeitet.

5. Generator nach Anspruch 4, **dadurch gekennzeichnet, dass** das Steuerprogramm dazu eingerichtet ist, das Abschaltsignal (sig) zu empfangen und in Reaktion auf das Abschaltsignal (sig) den Betrieb der Funktionsbaugruppe (12) zu beenden.

6. Generator nach einem der vorstehenden Ansprüche, **dadurch gekennzeichnet, dass** die Netzüberwachungseinrichtung (37) über einen Vollweggleichrichter (D1, D3, D5, D6) mit dem Netzspannungseingang (21) und ausgangsseitig mit einer Zeitgeberschaltung (47, 48, 49) verbunden ist.

7. Generator nach Anspruch 6, **dadurch gekennzeichnet, dass** die Zeitgeberschaltung (47, 48, 49) die Karenzzeitspanne (K) bestimmend ausgebildet ist.

8. Generator nach einem der vorstehenden Ansprüche, **dadurch gekennzeichnet, dass** die Zeitgeberschaltung (47, 48, 49) als retriggerbares Monoflop ausgebildet ist.

9. Generator nach einem der vorstehenden Ansprüche, **dadurch gekennzeichnet, dass** die Netzüberwachungseinrichtung (37) mindestens einen netzgetriggerten Impulsformer (44, 45, 46) aufweist, der dazu eingerichtet ist, für jede einen Triggerimpuls (TP) generierende Netzhalbwelle einen Ladeimpuls (LP) zu erzeugen, der einem RC-Glied mit einem Speicherkondensator (47) und einem diesem parallel geschalteten Entladewiderstand (48) zugeleitet ist, wobei das RC-Glied eine Zeitkonstante (τ) festlegt und wobei der Speicherkondensator (47) von jedem Ladeimpuls auf eine festgelegte Ladespannung (U_{L}) aufgeladen wird.

10. Generator nach Anspruch 9, **dadurch gekennzeichnet, dass** dem RC-Glied (47, 48) ein Schwellwertschalter (49) nachgeordnet ist, der eine festgelegte Schaltschwelle aufweist.

11. Generator nach Anspruch 10, **dadurch gekennzeichnet, dass** die Ladespannung, die Schaltschwelle und die Zeitkonstante (τ) so aufeinander abgestimmt sind, dass die Schaltschwelle erst nach Wegfall mehrerer Ladeimpulse (LP) erreicht wird.

12. Generator nach einem der vorstehenden Ansprüche, **dadurch gekennzeichnet, dass** die Stromversorgungseinheit (20) einen Netzgleichrichter (27) aufweist, der eingangsseitig an den Netzeingang (21) angeschlossen ist und einen Ausgang aufweist, an den eine Leistungsfaktor-Korrekturschaltung (PFC) angeschlossen ist.

13. Generator nach Anspruch 12, **dadurch gekennzeichnet, dass** die Leistungsfaktor-Korrekturschaltung (PFC) mit wenigstens einem Speicherkondensator (28) speisend verbunden ist, der Teil des Speichers (26) ist.

14. Generator nach Anspruch 13, **dadurch gekennzeichnet, dass** an den Speicherkondensator (26) ein Gleichspannungsumsetzer (DC/DC) angeschlossen ist, der ausgangsseitig wenigstens einen Speicherkondensator (36) aufweist, der Teil des Speichers (26) ist.

15. Generator nach Anspruch 14, **dadurch gekennzeichnet, dass** der Gleichspannungsumsetzer (DC/DC) potentialtrennend ausgebildet ist.
